(19) **Europäisches Patentamt**
**European Patent Office**
**Office européen des brevets**

(11) **EP 2 969 966 B1**

(12) **EUROPEAN PATENT SPECIFICATION**

(45) Date of publication and mention
of the grant of the patent:
**02.05.2018 Bulletin 2018/18**

(21) Application number: **14718178.8**

(22) Date of filing: **13.03.2014**

(51) Int Cl.:
**C02F 1/32** (2006.01)    **A61L 9/20** (2006.01)

(86) International application number:
**PCT/IL2014/050269**

(87) International publication number:
**WO 2014/141269 (18.09.2014 Gazette 2014/38)**

(54) **SYSTEM AND METHOD FOR INACTIVATION OF INFECTIOUS PANCREATIC NECROSIS VIRUS (IPNV) USING OPTIMIZED ULTRAVIOLET (UV) LIGHT**

SYSTEM UND VERFAHREN ZUR INAKTIVIERUNG DES INFEKTIÖSEN PANKREASNEKROSE-VIRUS MITHILFE VON OPTIMIERTEM ULTRAVIOLETT (UV)-LICHT

SYSTÈME ET PROCÉDÉ D'INACTIVATION DU VIRUS DE LA NÉCROSE PANCRÉATIQUE INFECTIEUSE (VNPI) À L'AIDE DE LUMIÈRE ULTRAVIOLETTE (UV) OPTIMISÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2013  US 201361788477 P**

(43) Date of publication of application:
**20.01.2016 Bulletin 2016/03**

(73) Proprietor: **Atlantium Technologies Ltd.**
**Beit-Shemesh 99100 (IL)**

(72) Inventors:
• **LICHI, Tovit**
**7053008 Gedera (IL)**
• **KERTSER, Michael**
**6086000 Bney Aish (IL)**
• **ROZENBERG, Ytzhak**
**5236704 Ramat Gan (IL)**

(74) Representative: **Pearl Cohen Zedek Latzer Baratz UK LLP**
**16 Upper Woburn Place**
**London WC1H 0BS (GB)**

(56) References cited:
**WO-A1-2007/113537     WO-A1-2008/059503**
**WO-A1-2010/071814     US-A1- 2008 206 095**
**US-A1- 2011 226 966**

• **HELGE LILTVED ET AL: "Inactivation of bacterial and viral fish pathogens by ozonation or UV irradiation in water of different salinity", AQUACULTURAL ENGINEERING, vol. 14, no. 2, 1 January 1995 (1995-01-01), pages 107-122, XP055119068, ISSN: 0144-8609, DOI: 10.1016/0144-8609(94)P4430-J**
• **LILTVED H ET AL: "High resistance of fish pathogenic viruses to UV irradiation and ozonated seawater", AQUACULTURAL ENGINEERING, ELSEVIER SCIENCE PUBLISHERS LTD, AMSTERDAM, NL, vol. 34, no. 2, 1 March 2006 (2006-03-01), pages 72-82, XP027941164, ISSN: 0144-8609 [retrieved on 2006-03-01]**

**Description**

**BACKGROUND**

Infectious Pancreatic Necrosis Virus (IPNV) is a common contaminant in water and other liquids in fish farms and is suspected of depleting populations of salmon species, such as, Norwegian and Chilean salmon. Ultraviolet (UV) light may be used to disinfect contaminated liquid to inactivate IPNV and thus, reduce the risk of illness. To effectively decontaminate liquid, IPNV may be inactivated to a degree greater than or equal to a log value of 3 ($\geq$ 99.9% inactivation) by radiating with a standard low pressure UV lamp at a wavelength of 254 nm and applying at least a UV dose of around 250 mJ/cm$^2$. Since the UV dose needed for inactivation is relatively high, standard UV lamps use relatively high amounts of electricity to achieve effective decontamination.

**SUMMARY**

Embodiments of the invention provide a method according to claim 1 for using a UV lamp "tuned" to optimize IPNV inactivation. In tests carried out according to embodiments of the invention, lamps having a wavelength above 260 nanometers (nm) and/or below 245nm were found to inactivate IPNV, e.g., between 3 and 70 times more than UV light at 254nm or more accurately at 253.7nm. The value of 254 nm, as used herein refers to the current commonly used industrial LP lamps, which emit at the wavelength of 253.7nm.Water transmission is better in the high range (e.g. above 260 nm) than the low range (e.g. below 245nm). Further, polychromatic (medium pressure) UV lamps substantially in a wavelength range between 260 and 400nm were discovered to inactivate IPNV using significantly less UV light or UV dose than monochromatic (low pressure) UV lamps emitting in 253.7nm.

In some embodiments of the invention, a liquid containing IPNV may be illuminated with a spectrally optimized medium pressure UV light in a UV dose level of less than approximately 50% compared to a UV dose level of a low pressure lamp emitting at 253.7nm to produce the same level of log inactivation of IPNV. Accordingly, spectrally optimized medium pressure lamp requires less electricity to produce the same level of inactivation level. The lower UV dose level the less electricity required. Optimized IPNV customized lamps are lamps customized and designed based on the response sensitivity of the IPN virus. Based on the response sensitivity of the IPN virus, higher pressure optimized MP lamps may contribute more to a cumulative intensity than the lower pressure MP lamp. Embodiments of the invention include a MP lamp with pressure higher than 1.6 bar and preferably, higher than 4 or 5 bar so as to match the spectral response of the IPN virus to UV light.

In some embodiments, a UV lamp "tuned" to optimize IPNV inactivation may be a medium pressure polychromatic UV lamp that emits UV light substantially in a wavelength range of between 260 and 400nm. In some embodiments, the UV lamp may be a medium pressure UV lamp that emits UV light substantially in a wavelength range of between 260 and 300nm. The "tuned" medium pressure polychromatic UV lamp may have a pressure above 1.6 bar, for example above 3 bar, above 5 bar, above 6 bar etc. In other embodiments, the UV lamp may be a monochromatic lamp that emits UV light having a single wavelength in the range of 260-400nm and preferably, in the range of 260-300nm.

In some embodiments, a UV lamp "tuned" to optimize IPNV inactivation may be a medium pressure polychromatic UV lamp that emits UV light substantially in a wavelength range of between 200 and 245nm. In some embodiments, the UV lamp may be a medium pressure UV lamp that emits UV light substantially in a wavelength range of between 200 and 220nm. The "tuned" medium pressure polychromatic UV lamp may have a pressure above 1.6 bar, for example above 3 bar, above 5 bar, above 6 bar etc. In other embodiments, the UV lamp may be a monochromatic lamp that emits UV light having a single wavelength in the range of 200-245nm and preferably, in the range of 200- 220nm.

Both polychromatic and monochromatic UV lamp in both the upper and lower wavelength ranges are referred to herein as "IPN customized UV lamp". The IPN customized UV lamps according to embodiments of the invention may be composed of pure silica synthetic quartz (PS)

**BRIEF DESCRIPTION OF THE DRAWINGS**

The subject matter regarded as the invention is particularly pointed out and distinctly claimed in the concluding portion of the specification. The invention, however, both as to organization and method of operation, together with objects, features, and advantages thereof, may best be understood by reference to the following detailed description when read with the accompanying drawings in which:

Figs. **1** and **2** schematically illustrate systems for disinfecting a liquid containing in accordance with embodiments of the invention;
Fig. **3** schematically illustrates a system for determining the biological response of IPNV to UV light in accordance with embodiments of the invention;

Fig. **4** is a graph that plots the correlation between UV doses for low pressure UV light, respectively, and the inactivation of IPNV, not in accordance with embodiments of the invention;

Fig. **5** is a graph of the relative spectra of MP UV lamps at different pressures (e.g., defined by mercury (Hg) bars);

Fig. **6** is a graph of the cumulative intensity of the MP lamps with different pressures;

Fig. **7** is a graph of the IPN spectral response curve, normalized at 254nm;

Fig. **8** is a graph of the cumulative intensity of the MP lamps with different pressures with respect to the IPN spectral response;

Fig. **9** is a summary table listing the total radian power and normalized radian power of a PS (synthetic quartz) MP lamp and a regular quartz MP lamp; and

Fig. **10** is a graph of the spectra of a PS (synthetic quartz) MP lamp (violet), and the regular quartz (blue).

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

## DETAILED DESCRIPTION

In the following description, various aspects of the present invention will be described. For purposes of explanation, specific configurations and details are set forth in order to provide a thorough understanding of the present invention. However, it will also be apparent to one skilled in the art that the present invention may be practiced without the specific details presented herein. Furthermore, well known features may be omitted or simplified in order not to obscure the present invention.

Unless specifically stated otherwise, as apparent from the following discussions, it is appreciated that throughout the specification discussions utilizing terms such as "processing," "computing," "calculating," "determining," or the like, refer to the action and/or processes of a computer or computing system, or similar electronic computing device, that manipulates and/or transforms data represented as physical, such as electronic, quantities within the computing system's registers and/or memories into other data similarly represented as physical quantities within the computing system's memories, registers or other such information storage, transmission or display devices.

In some embodiments of the invention, a UV lamp is provided that is optimized to inactivate IPNV. The UV lamp may be a medium pressure UV lamp that emits UV light substantially in a wavelength range of between 260 and 400nm. In some embodiments, the UV lamp may be a medium pressure UV lamp that emits UV light substantially in a wavelength range of between 260 and 300nm.

Disinfection systems may irradiate liquids with UV light to reduce or inactivate IPNV. UV lamps may be monochromatic or polychromatic. Monochromatic lamps emit a single wavelength of UV light (for example, 280 nanometers (nm)). Throughout the specification, only the commonly used monochromatic UV lamps that emit at around 254nm are referred to as "low pressure" (LP) UV lamps. Polychromatic lamps emit multiple wavelengths of UV light, for example, in discrete increments or in a continuous broad band range (e.g., 260-300 nm), and may be referred to as "medium pressure" (MP) UV lamps. In tests carried out according to embodiments of the invention, optimally customized medium pressure (MP) UV lamps were discovered to inactivate IPNV at significantly greater rates than standard low pressure (LP) UV lamps configured to emit at 253.7nm. Since the standard LP and customized MP UV lamps inactivate IPNV at different rates, the proper UV intensity and UV exposure time, i.e., the "UV dose," to decontaminate liquid may differ for each of the lamps.

Measuring the UV dose may be simple for a LP UV lamp (e.g., using equation $D_{CB}$ defined below) since illumination exposure parameters may be easily measured for the single wavelength of the monochromatic UV light. However, this calculation may not be used for polychromatic light. MP UV lamps, which radiate light over multiple wavelengths, include some wavelengths that actively reduce IPNV, and other wavelengths that are innocuous or less effective at reducing IPNV. To accurately determine the intensity of MP light, the intensity may be measured separately at each of the active wavelengths, a generally difficult task. Currently, only the UV dose to disinfect IPNV with a standard LP UV lamp emitting at 253.7nm is known, while the UV dose for MP light remains unknown.

To accurately calculate the intensity or UV dose of MP UV light, which may be difficult to measure directly, embodiments of the invention may measure the UV dose using an LP UV light that gives an equivalent (of near equivalent) reduction in microorganisms. Such an MP UV dose may be referred to as a "reduction equivalent" UV dose (RED) since it is not measured, but determined to be equivalent to a LP UV dose that achieves a similar reduction. In one embodiment, to determine the reduction equivalent UV dose for MP radiation of IPNV, the LP UV dose may be measured, not for IPNV, but for another control organism, such as, male specific-2 bacteriophage (MS2). The control organism may have a known correlation between the LP UV doses and the reduction equivalent MP UV doses used to inactivate the same amounts of the control organism. MS2 is inactivated in equivalent UV doses by LP and MP UV light, although other control organisms with non-equivalent LP and MP UV doses may also be used, as long as the correlation between their LP and MP UV doses is known or measurable.

In one example, the MP UV dose used to achieve at least a 3 log ($\geq$ 99.9%) reduction of IPNV was determined to be at least 50% lower compared to a UV dose level of a low pressure lamp emitting at 253.7nm. (LP UV dose) For comparison, the LP UV dose used to achieve the same reduction in IPNV is approximately 120-246 mJ/cm$^2$. Thus, MP lamps provide the same reduction as LP lamps, but with such a significantly smaller UV dose, for example, at least 3-5 times less than LP lamps. Whereas there is substantially no difference between radiating MS2 with MP and LP UV light, such a significant difference between radiating IPNV with MP and LP UV light provides a unique and unexpected result.

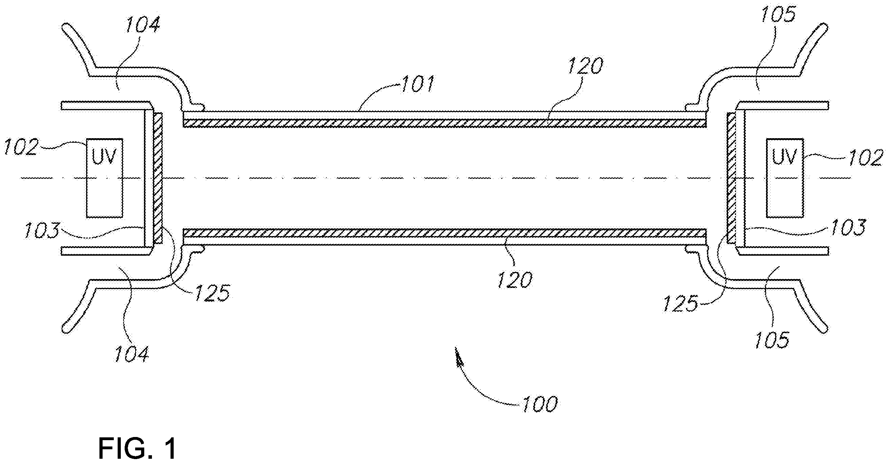

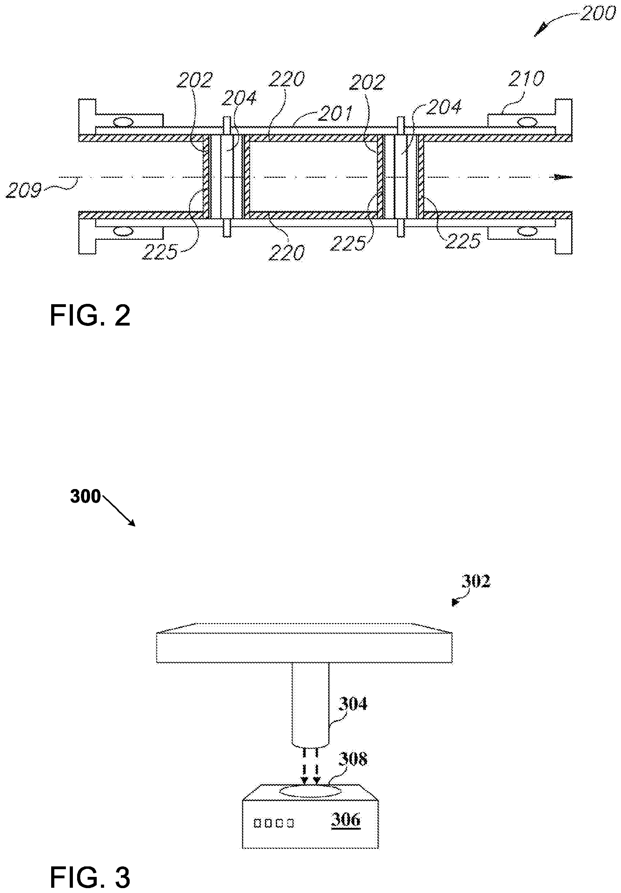

Reference is made to Figs. **1** and **2,** which schematically illustrate systems **100** and **200** for disinfecting a liquid containing IPNV in accordance with embodiments of the invention. Fig. **1** includes UV sources **102** positioned externally to conduit **101** and Fig. **2** includes UV sources **204** positioned internally to conduit **201** within the cavity of the channel, although any arrangement or combination of internal and/or external UV sources may be used.

In Fig. **1,** system **100** may include a tube, channel or conduit **101** to carry flowing liquid to be disinfected and one or more external UV sources **102** to illuminate and to disinfect the liquid within conduit **101**. Conduit **101** may have an inlet **104** to receive the liquid, and an outlet **105** to discharge the liquid. Conduit **101** may be made, at least partially, of a UV transparent material, such as glass or quartz. System **100** may include one or more windows **103** which may be made of UV transparent material, such as glass or quartz and may, for example, be coated with an anti-deposit layer **125..**

UV sources **102** may illuminate or irradiate the liquid when flowing in conduit **101** to inactivate contaminants, such as, IPNV. In some embodiments, the liquid within conduit **101** may act as a waveguide and at least part of the light, for example, at least half of the emitted UV intensity, may be totally-internally reflected at the interface of the UV-transparent conduit **101** and the surrounding air. UV sources **102** may include one or more IPN customized UV lamp, for example medium pressure UV sources, adapted to emit polychromatic light at multiple (discrete or continuous) wavelengths (e.g., in a range throughout the germicidal UV range and beyond). UV source 104 may comprise pure silica synthetic quartz (PS) UV sources 102 may emit UV light substantially in a wavelength range of between 200 and 245nm. In some embodiments, the UV lamp may be a medium pressure UV lamp that emits UV light substantially in a wavelength range of between 200 and 220nm. The "tuned" medium pressure polychromatic UV lamp may have a pressure above 1.6 bar, for example above 3 bar, above 5 bar, above 6 bar etc. In other embodiments, UV source **102**may be a monochromatic lamp that emits UV light having a single wavelength in the range of 200-245nm and preferably, in the range of 200-220nm.

UV sources **102** may be set to emit UV light at a fixed UV dose predetermined (e.g., in the testing phase of Fig. **3**) to inactivate IPNV by an optimal degree. For example, medium pressure UV sources **102** may illuminate liquid with a UV dose of less than or equal to 80 mJ/cm$^2$ to achieve an inactivation of IPNV by at least a 3 log values (99.9%).

In Fig. **2,** system **200** may include a conduit **201** to carry flowing liquid to be disinfected and one or more internal UV sources **204** to illuminate and to disinfect the liquid within conduit **101.** An illuminating surface of each UV source **204** may be substantially perpendicular to a longitudinal axis of symmetry **209** of conduit **201,** such that the direction of illuminating rays is parallel to longitudinal axis of symmetry **209..** UV source 204 may comprise pure silica synthetic quartz (PS). UV source 204 may emit UV light substantially in a wavelength range of between 200 and 245nm. In some embodiments, the UV lamp may be a medium pressure UV lamp that emits UV light substantially in a wavelength range of between 200 and 220nm. The "tuned" medium pressure polychromatic UV lamp may have a pressure above 1.6 bar, for example above 3 bar, above 5 bar, above 6 bar etc. In other embodiments, UV source **102** may be a monochromatic lamp that emits UV light having a single wavelength in the range of 200-245nm and preferably, in the range of 200-220nm. Each UV source **204** may be positioned in a UV-transparent sleeve **202.** Sleeve 202 may comprise pure silica synthetic quartz (PS) .

In the examples shown in Figs. **1** and **2,** systems **100** and **200** are flow systems that disinfect liquid as it passes through conduits **101** and **201,** respectively, although systems **100** and **200** may be configured in any other arrangement, such as, a movable UV source passing through a closed container of liquid, a UV source with sufficient surface area to irradiate an entire liquid sample so that no relative motion is needed to expose all the liquid, etc..

Reference is made to Fig. **3,** which schematically illustrates a bio-dosimetry system **300** for determining the biological response of IPNV to UV light in accordance with embodiments of the invention.

System **300** may include a collimated beam apparatus **302** with a UV source **304** and a detector **306** which may include a magnetic stirrer. Samples **308** of liquid contaminated with known quantities of microorganisms are positioned between the UV source **304** and detector **306** to test their response to UV doses of low and/or medium pressure light from UV sources **304.** Samples **308** may include aliquots of mixed cultures, e.g., placed in Petri dishes, including IPNV and a control organism, such as, MS2, tested separately.

The IPNV sample **308** may be exposed to medium pressure UV source **304** for various exposure conditions, such as, various intensities and/or exposure times, and collimated beam apparatus **302** may measure the concentration of inactive (or active) IPNV for each of these exposures. Collimated beam apparatus **302** may measure the IPNV inactivation concentrations corresponding to each set of exposure conditions. Exposures conditions above a certain intensity and/or duration may inactivate IPNV by an optimal concentration (e.g., by at least log 3). Although the exposure conditions are known, the corresponding medium pressure UV doses may not be easily calculated directly due to the spectral spread of polychromatic light.

To deduce the medium pressure UV doses, the UV dose-response may be tested for a control organism with a known correlation between MP and LP UV doses.

For example, MS2 inactivates by substantially similar amounts when exposed to the same MP and LP UV doses. The control organism may be exposed to a variety of medium pressure UV light to determine a correlation between IPNV and the control organism inactivation concentrations for the same exposure settings. The control organism may also be exposed to a variety of low pressure UV light to determine a correlation between LP UV doses and inactivation concentrations of the control organism.

The correlation between IPNV and the control organism inactivation concentrations for each set of MP exposure conditions may be used to correlate MP exposure conditions (linked to IPNV inactivation concentrations) and LP UV doses or their same reduction equivalent MP UV doses (linked to MS2 inactivation concentrations). Accordingly, the MP UV doses for IPNV may be deduced from easily measurable LP UV doses for the control organism. The optimal range of MP UV doses for IPNV may include the subset of those doses that correspond to exposure conditions that inactivate IPNV by an optimal degree (e.g., a log 3 reduction).

Tests were conducted according to embodiments of the invention using a low pressure collimated beam apparatus (LP-CBA) including a low pressure UV lamp measuring the LP UV dose-response for the control organism and IPNV (relating LP UV dose to log inactivation) and, similarly, optimized medium pressure collimated beam apparatus (MP-CBA) including a medium pressure UV lamp measuring the MP UV dose-response for the control organism and IPNV (relating medium pressure exposure settings to log inactivation). Each of the medium and low pressure CBA tests may be preformed, in parallel (e.g., the MS2 and IPNV samples were exposed to the same light at the same exposure time), for the control organism and IPNV to calculate their UV dose responses to the same exposure conditions. The log-inactivation values of IPNV from the MP-CBA were input into the laboratory derived-UV dose-response relationship (CBA test) of the control organism to estimate the reduction equivalent dose (RED) of IPNV delivered by the MP-CBA.

The UV dose calculation spectral respond tests is based on tests described in A. Lakretz et al. 2010 (Anat Lakretz; Eliora Z. Ron; Hadas Mamane; Biofouling control in water by various UVC wavelengths and doses; Biofouling Vol. 26, No. 3, April 2010, (57-267) with slight modifications as follows: The integrated average irradiance between 200 and 300 nm is calculated according to Bolton and Linden (2003) [Bolton JR, Linden KG. 2003. Standardization of methods for fluence (UV dose) determination in bench-scale UV experiments. J Environ Eng ASCE 129:209-215] using the spectral incident irradiance obtained from a calibrated spectroradiometer (USB4000, Ocean Optics) placed in the same x, y position as the center of the crystallization dish and at the surface of the liquid suspension, the water spectral absorbance obtained via a UV-Vis spectrophotometer (Secoman Uvikon xs), the reflection at the sample surface and the measured Petri factor for the dish. The UV influence is calculated by multiplying the average irradiance with exposure time. In addition, band-pass (BP) filters placed in the polychromatic light path will be used to transmit a well-defined band of light from the polychromatic MP light source at a central wavelength of 220, 239, 254, 260 and 280 nm with an average full width at half maximum (FWHM) of 20-27.5 nm and minimum peak transmittance ranges between 12 and 15% (Andover Corporation, NH, USA). The transmission curves for the BP filters, were performed with a UV-Vis spectrophotometer (Cary Bio100, Varian, Inc., Palo Alto, CA, USA) for absorbance measurement, equipped with a 150 mm diameter IS attachment (Diffuse Reflectance accessory (DRA)-CA-3330, Labsphere, NH, USA). The filter was placed in a holder at the sample transmission port of the integrating sphere. The actual average irradiance, to which the IPNV are exposed when the filters are used, is obtained by multiplying (weighting) the spectral incident irradiance (measured without filters) by the bandwidth at each wavelength (spectral transmittance in percentage), taking into consideration the water spectral absorbance, petri factor and the water reflection.

Different strains of IPNV, American Type Culture Collection (ATCC) #VR-1318 isolated from Trout and ATCC #VR-1320 isolated from Pike fry (Esox lucius), were tested to demonstrate the common response of different forms of IPNV to medium pressure UV doses. Both strains of IPNV were propagated in cell cultures including Blugill Fin cell (BF-2) and Chinook Salmon Embryo cell (CHSE-214) cultures. Cell cultures were grown in Minimum Essential Medium Eagle (MEM-Eagle) formulations with non-essential amino acids supplemented with 10% New-born Calf Serum (NBCS), 2mM L-glutamine, 5% Pen/ Strep solution and maintained in a 22-24°Celcius (°C) CO2 free incubator. Confluent cultures were infected with IPNV and incubated for 4-6 days at 15-16°C. Once extensive cytopathic effect (CPE) was evident in the cells, the medium was collected, aliquoted and maintained at -70°C for further use. Cells were seeded in 96 well plates, for CHSE-214 $5\times10^4$ cells/well and for BF-2 - $8\times10^4$ cells/well.

Virus samples were serially 10 fold diluted up to $10^{-8}$ dilutions and used to infect cell monolayers with each dilution assayed in triplicates. Infected cultures were cultivated MEM-Eagle with non-essential amino acids supplemented with 10% NBCS, 2mM L-glutamine and 20mM Hepes, at 15-16°C. Four days after infection, cells were fixed (with formaldehyde 2%) and stained for 10 min with 1.5% Genetian violet. 50% Tissue Culture Infective Dose (TCID50) titer was determined, for example, according to methods described in Reed, L.J., & Muench, H. (1938). L. J. Reed and H. Muench; A Simple Method of Estimating Fifty Per Cent Endpoints; The American journal of Hygiene Vol27 May 1938 No. 3 493-497. The control microorganism used was MS2 including a culture of male specific-2 bacteriophage (ATCC 155597-B1) and E.coli MS2 bacteriophage host (ATCC 155597). MS2 stocks were prepared and stored, for example, according to the procedure outlined in the Ultraviolet Disinfection Guidance Manual 2006 ("ULTRAVIOLET DISINFECTION GUIDANCE MANUAL" EPA 815-D-03-00 7April, 2006.)

The bacteriophage titer was checked once immediately after preparation, and again within 24 hours prior to the experiment. The collimated beam apparatus was operated, for example, according to the procedure outlined in the Ultraviolet Disinfection Guidance Manual. Germicidal light intensity and Petri factors were measured using OPHIR UV detector. The UV transmission (UVT) at 254 nm of the viral suspension was measured by a spectrophotometer in a 1 cm quartz cuvette.

The LP UV dose (namely, the US dose of a law pressure lamp emitting at 253.7nm), $D_{CB}$, may be calculated according to exposure conditions (e.g., measured ultraviolet transmitting (UVT) of the microbial suspension and UV intensity of low pressure lamp). The LP UV dose, $D_{CB}$, may be calculated separately for each CBA test, for example, as follows:

$$D_{CB} = E_i P_f (1-R) \frac{L(1-10^{-ad})}{(d-L)ad \ln(10)} t$$

where:

$D_{CB}$ = UV dose (mJ/cm$^2$)
$E_i$ = Average UV irradiance (measured before and after irradiating the sample) (milliwatts (mW)/cm$^2$)
$P_f$ = Petri factor (unit-less)
$R$ = Reflectance at the air-water interface at the irradiating wavelength (e.g., 254 nm) (unit-less)
$L$ = Distance from the lamp centerline to the suspension surface (cm)
$d$ = Depth of the suspension (cm)
$a$ = UV absorption coefficient (base 10) of the suspension at the irradiating wavelength e.g., 254 nm) (cm$^{-1}$)
$t$ = Exposure time (seconds)

LP UV doses were tested in a range of 0-120 mJ/cm$^2$ (0, 20, 40, 60, 80, 100, 120, mJ/cm$^2$) for MS2 bacteriophage and 0-250 mJ/cm$^2$ (0, 30, 60, 120, 250 mJ/cm$^2$) for IPNV. The UV lamp used in the LP-CBA was a 15 watt (W) low-pressure germicidal lamp (emitting at 253.7nm) and the UV lamp used in the MP-CBA was a 1 kilowatt (kW) optimized medium-pressure IPV customized lamp. The samples were illuminated using two lamp powers: 100% and 25%. 20 ml samples of each viral suspension were illuminated with LP-CBA and with MP-CBA in Petri dishes (of 50 cm$^2$ surface area, 1 cm sample depth) with slow stirring. The IPNV suspension was illuminated and kept on ice during testing.

The samples were illuminated with the maximum dose to test for possible harmful effect of the solution to the IPNV. IPNV was added to the samples immediately after the illumination in the same concentration as added to the non-UV illumination sample. These samples are referred to as "medium with IPNV" in tables 1-3. Samples without IPNV were illuminated with the maximum dose to test for possible harmful effect of the solution to the cells. These samples are referred to as "medium w/o IPNV" in tables **1-3.**

The concentration of each of the samples was determined using an MS2 enumeration procedure (e.g., the Pour Plate method) and IPNV plaque assay/end point titration procedure. The log inactivation may be computed, for example, as follows:

$$\log 10 \, (No/N)$$

where:

$No$ = Concentration of microorganisms prior to UV light exposure.
$N$ = Concentration of microorganisms present after UV light exposure

The experimental results were computed as a UV dose to log inactivation function, and a trend line equation was determined for each LP-CBA experiment.

The reduction equivalent UV dose value of MP-CBA was determined by comparing the MS2 bacteriophage's log inactivation achieved by the LP-CBA to the equal log inactivation achieved by the MP-CBA under specific condition (MP-UV exposure time, suspensions UVT at 254 nm, UV lamp power, etc.). The RED value required for inactive IPNV was determined by comparing the MS2 bacteriophage's MP-UV exposure time (with specific known RED) to MP-UV exposure time of IPNV under specific exposure conditions.

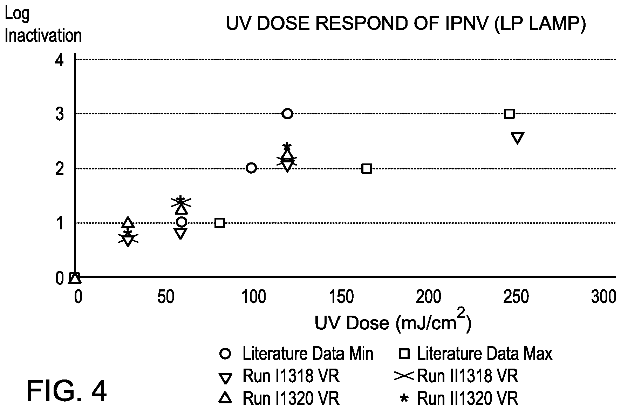

Reference is made to Fig. **4,** which is a graph that plots correlations between UV doses and the inactivation of IPNV, not in accordance with embodiments of the invention. Fig. **4** shows results for low pressure UV doses (listed in table **1**) The results for low pressure UV doses (in Fig. **4**) are from tests conducted not accordance with embodiments of the invention and from literature data (e.g., H. Liltved et al.; High resistance of fish pathogenic viruses to UV irradiation and ozonated seawater; Aquacultural Engineering 34 (2006) 72-82).

Table **1** lists results of the IPNV inactivation by UV light irradiated with low pressure (LP) lamps. IPNV inactivation results are measured for two strains of IPNV, ATCC no. VR1318 (isolated from Trout) and ATCC #VR-1320 (isolated from Pike fry), in two independent tests, "run 1" and "run 2," to verify results. Only samples with > 25 PFU/ml may be considered viable. Disinfection may be considered optimal for a log inactivation greater than or equal to 3.

Table 1: LP lamp UV dose (in mJ/cm2) for IPNV inactivation

| LP UV dose (mJ/cm$^2$) | ATCC no. VR1318 | | ATCC no. VR1320 | |
| | Run I | Run II | Run I | Run II |
| | Log. Inact. | Log. Inact. | Log. Inact. | Log. Inact. |
| --- | --- | --- | --- | --- |
| 0 | 0.00 | 0.00 | 0.00 | 0.00 |
| 30 | 0.76 | 0.72 | 1.00 | 0.84 |
| 60 | 0.85 | 1.36 | 1.25 | 1.40 |
| 120 | 2.11 | 2.10 | 2.25 | 2.40 |
| 250 | 2.60 | >5.2* | >3.7* | >3.5* |
| Medium with IPNV | -0.08 | NA | 0.29 | NA |
| Medium w/o IPNV | NA | NA | 0.00 | 0.00 |
| * Invalidated values | | | | |

In tests conducted in accordance with embodiments of the invention, no harmful effect to the cell and to the IPNV were detected in the illuminated solutions. No significant difference was observed between the responses of the two *IPNV* strains, ATCC no. VR1318 and ATCC #VR-1320, when illuminated with UV light. The optimized MP UV lamp was found to be more effective for inactivating IPNV than was the LP UV lamp. In the set of CBA tests performed with the optimized medium pressure (MP) lamp for achieving an inactivation level of the IPNV between 0.2-3.3 logs reduction it was found that the optimized medium pressure lamp (MP) was more efficient by 2.2-8.6 time relative to the LP lamp

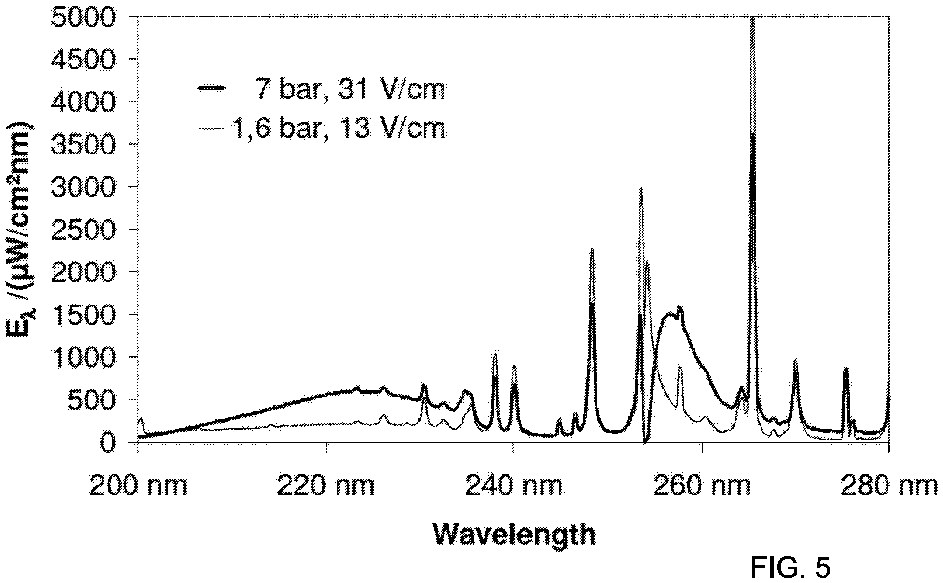

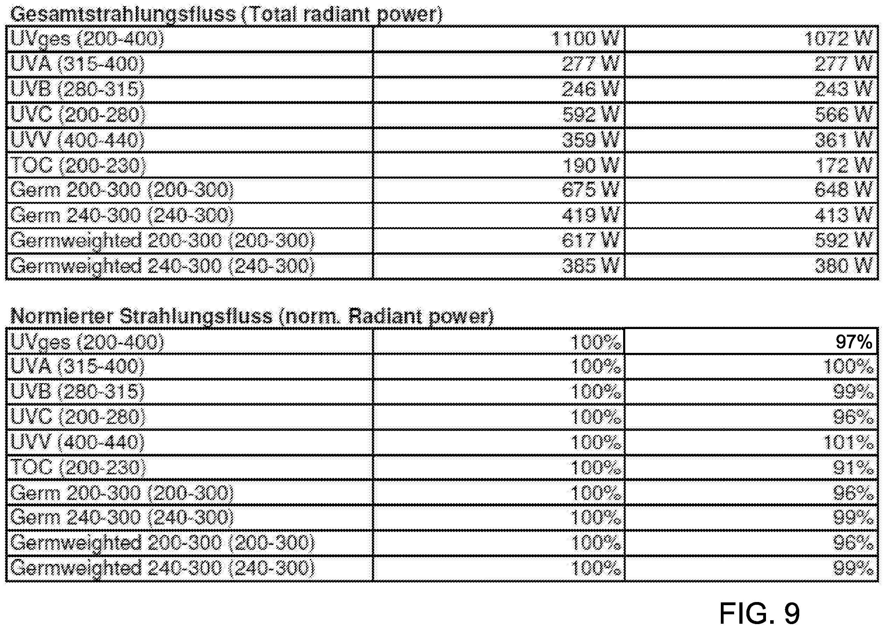

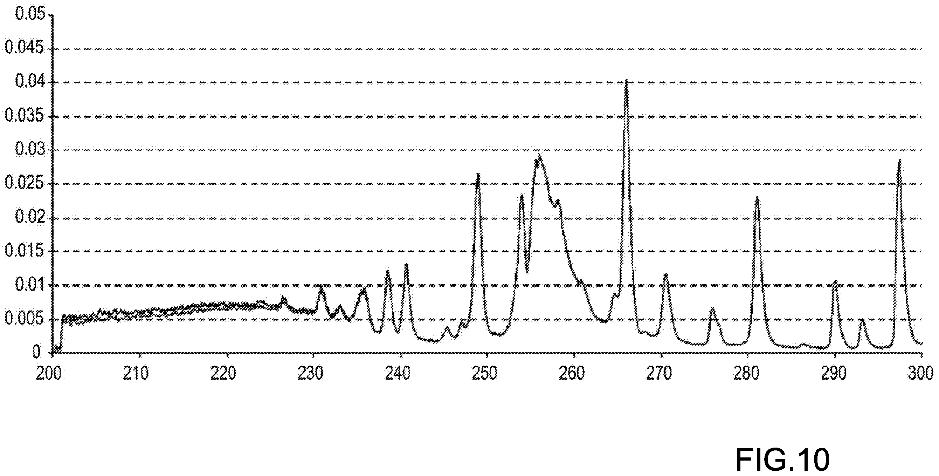

Reference is made to Fig. **5,** which is a graph of the relative spectra of MP UV lamps at different pressures (e.g., defined by mercury (Hg) bars). This figure shows the difference in the relative contributions of different MP Hg UV lamps to the UV effect on IPN. Contributing factors may be taken into account: (1) Variation of pressure (e.g., measured by mercury (Hg) bar) in MP lamp will cause enhancement of the specific spectral bands and (2) the material of the envelope and (3) the composition of the materials in the lamp. (1) Pressure tuning is discussed in reference to Figs. 5, **6** and **8.** (2) The material of the envelope is discussed in reference to Figs. **9** and **10.** A lamp envelope made of substantially pure silica synthetic quartz which enables higher transparency at 200-230nm spectral range and by this the lamps emits more UV light at the range of 200-240nm, the amount of light can be about 9% higher. (3) Changing the lamps internal chemistry e.g. by doping, may also increase IPN inactivation since the materials of the lamp affect spectral transmission of UV light. For example, the lamps may be composed of mercury e.g. doped with a metal halide additive such as Iron Halide. Such doping may cause the lamp to have higher radiation e.g. in the range of 200-230 and 260-300nm. In addition, some embodiments may further use "electrode-less" lamps to enable more flexibility in using different lamp chemistry.

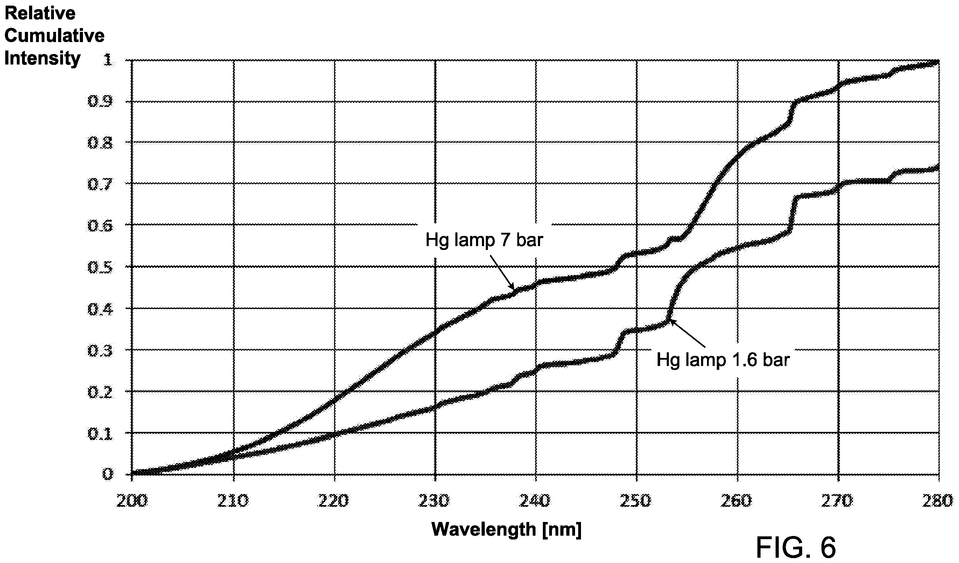

Reference is made to Fig. **6,** which is a graph of the cumulative intensity of the MP lamps with different pressures. The upper line shows the cumulative intensity of a mercury lamp having 7 Hg bars pressure and the lower line shows the cumulative intensity of a mercury lamp having 1.6 Hg bars pressure. The intensities in these figures are normalized.

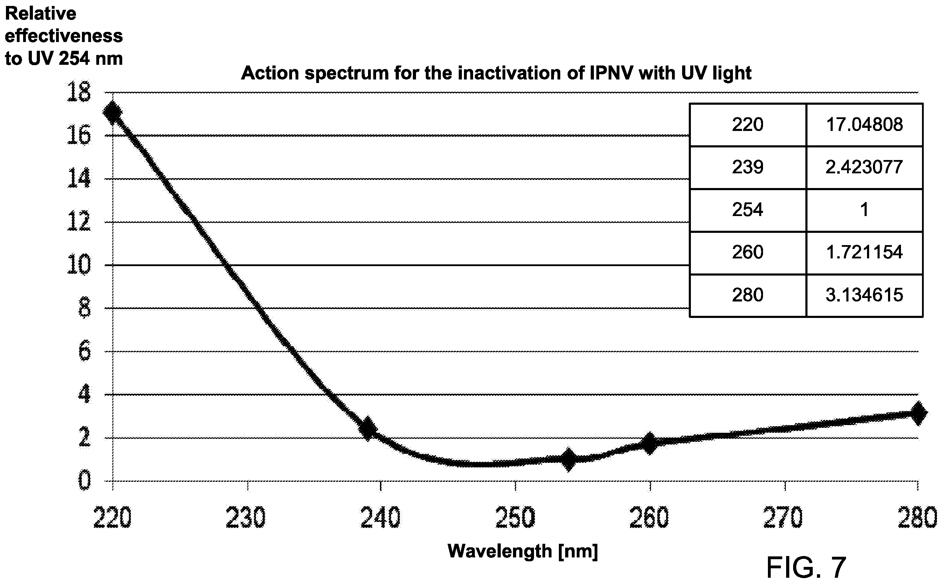

Reference is made to Fig. **7,** which is a graph of the IPN spectral response curve, normalized at 254nm. This graph shows that IPN is inactivated at a minimum around 254nm. Inactivation increases exponentially by both raising and lowering the wavelength to higher and lower ranges. At the lower range (below 254nm), inactivation of IPN increases by 17 times. At the higher range (above 254nm), inactivation of IPN increases by 3 times. The higher range may be preferred in some embodiments because of increased water transmission of UV light at higher wavelengths.

For example, IPN is 70% more sensitive in the wavelength of 260nm, 300% more sensitive in a wavelength of 280nm and 1700% in a wavelength of 220nm. Accordingly, a lamp may be used that emits UV light above 260nm so that the needed dose will be 70% lower than one that is using a lamp of 254nm. A UV system according to embodiments of the invention may include a lamp that emits UV light at a wavelength of approximately 220nm to be 1700% more efficient than a system that is based on UV lamp emit UV light at a wavelength of 254nm.

Another important parameter associated with the performance of UV system is the water transmission to UV light (UVT). Water transmission decreases as the wavelength spectrum decreases. For example, for the water of Ein Gedi illuminated at 220nm, after 1 cm the amount of remaining intensity was 35%. At 260nm, after 1 cm, the amount of remaining intensity was 90%. That is, 90% of the photons were still available for inactivating the IPN. Accordingly, a higher range of wavelengths may be preferred.

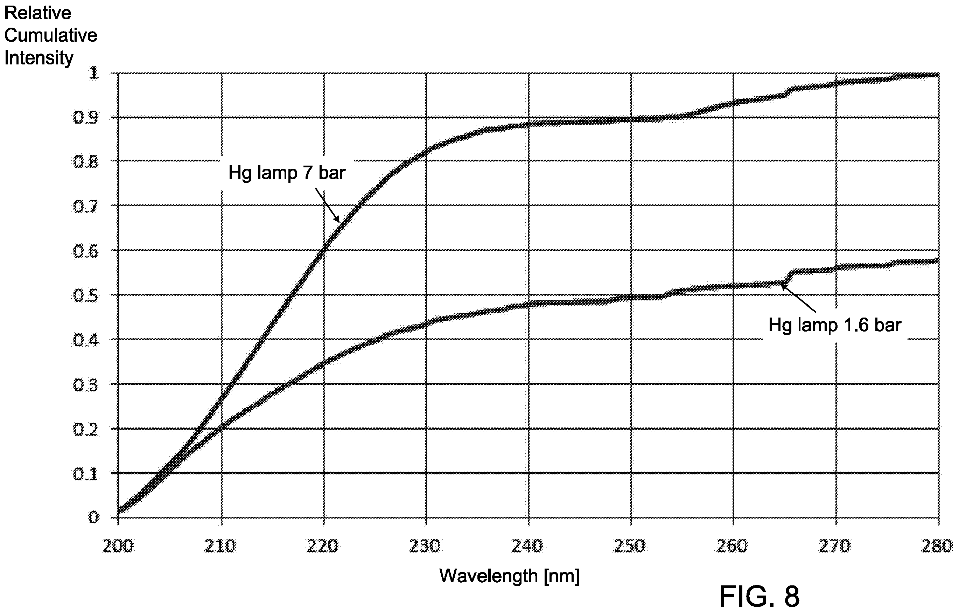

Reference is made to Fig. 8, which is a graph of the cumulative intensity of the MP lamps with different pressures with respect to the IPN spectral response. The upper line shows the cumulative intensity of a mercury lamp having 7 Hg bars pressure and the lower line shows the cumulative intensity of a mercury lamp having 1.6 Hg bars pressure. As shown in Fig. 6, a higher pressure MP lamp may contribute approximately 40% more to the cumulative intensity than the lower pressure MP lamp. Embodiments of the invention include a MP lamp with pressure higher than 1.6 bar and preferably, higher than 4 or 5 bar, for example, 7 bar.

Reference is made to Fig. 9, which is a summary table listing the total radian power and normalized radian power of a PS (synthetic quartz) MP lamp and a regular quartz MP lamp.

Reference is made to Fig. 10, which is a graph of the spectra of a PS (synthetic quartz) MP lamp (violet), and the regular quartz (blue). Fig. 10 shows that the difference in the IPN response sensitivity to the 200-230nm wavelength range (TOC(200-230nm) PS to Reg) may reach up to 9%, contributing to the overall cumulative germicidal intensity. Embodiments of the invention may include a lamp composed of pure silica synthetic quartz. Such a lamp may have a relatively high transparency at 200-230nm spectral range.

Different embodiments are disclosed herein. Features of certain embodiments may be combined with features of other embodiments; thus certain embodiments may be combinations of features of multiple embodiments.

The foregoing description of the embodiments of the invention has been presented for the purposes of illustration and description. It is not intended to be exhaustive or to limit the invention to the precise form disclosed. It should be appreciated by persons skilled in the art that many modifications, variations, substitutions, changes, and equivalents are possible in light of the above teaching.

**Claims**

1. A method of inactivation of Infectious Pancreatic Necrosis Virus (IPNV) comprising:

    illuminating a liquid containing IPNV with a ultraviolet (UV) light emitted from a medium pressure mercury UV lamp tuned to optimize IPNV inactivation,

    wherein the UV light being substantially in a wavelength range between 260 and 400nm or between 200 and 245nm and the lamp exhibits a pressure greater than 1.6 bar .

2. The method of claim 1, wherein illuminating the liquid containing the IPNV comprises illuminating the liquid with a UV dose of less than or equal to 80 mJ/cm$^2$ for 99.9% inactivation of the IPNV.

3. The method of claim 1, wherein the pressure of the lamp is greater than 3 bar.

4. The method of claim 1, wherein the pressure of the lamp is greater than 6 bar.

5. The method of claim 1, wherein the UV lamp is composed of pure silica synthetic quartz.

6. The method of claim 1, wherein the UV lamp is doped with a metal halide additive.

**Patentansprüche**

1. Ein Verfahren zur Inaktivierung des infektiösen Pankreasnekrose-Virus (IPNV), umfassend:

das Bestrahlen eines Flüssigkeit enthaltenden IPNV mit einer ultravioletten (UV) Strahlung, die von einer Quecksilber-Mitteldruck-UV-Lampe abgegeben wird, die eingestellt ist, um die IPNV-Inaktivierung zu optimieren, wobei die UV-Strahlung im Wesentlichen in einem Wellenlängenbereich zwischen 260 und 400 nm oder zwischen 200 und 245 nm liegt und die Lampe einen Druck höher als 1,6 bar aufweist.

2. Das Verfahren nach Anspruch 1, wobei das Beleuchten des Flüssigkeit enthaltenden IPNV das Bestrahlen der Flüssigkeit mit einer UV-Dosis von weniger als oder gleich 80 mJ/cm$^2$ für 99,9% Inaktivierung des IPNV umfasst.

3. Das Verfahren nach Anspruch 1, wobei der Druck der Lampe höher als 3 bar ist.

4. Das Verfahren nach Anspruch 1, wobei der Druck der Lampe höher als 6 bar ist.

5. Das Verfahren nach Anspruch 1, wobei die UV-Lampe aus reinem synthetischem Silikatquarz besteht.

6. Das Verfahren nach Anspruch 1, wobei die UV-Lampe mit einem Metall-Halogen-Additiv dotiert ist.


## Revendications

1. Un procédé d'inactivation du virus de la nécrose pancréatique infectieuse (VNPI) comprenant :

l'irradiation d'un liquide contenant le VNPI avec une lumière ultraviolette (UV) émise à partir d'une lampe UV au mercure à pression moyenne réglée pour optimiser l'inactivation du VNPI, dans lequel la lumière UV se trouve sensiblement dans une plage de longueur d'onde comprise entre 260 et 400 nm ou 200 et 245 nm et la lampe présente une pression supérieure à 1,6 bar.

2. Procédé selon la revendication 1, **caractérisé en ce que** l'irradiation du liquide contenant le VNPI comprend l'irradiation du liquide avec une dose UV inférieure ou égale à 80 mJ/cm$^2$ pour l'inactivation à 99,9 % du VNPI.

3. Procédé selon la revendication 1, dans lequel la pression de la lampe est supérieure à 3 bars.

4. Procédé selon la revendication 1, dans lequel la pression de la lampe est supérieure à 6 bars.

5. Procédé selon la revendication 1, dans lequel la lampe UV est composée de quartz synthétique en silice pure.

6. Procédé selon la revendication 1, dans lequel la lampe UV est dopée avec un additif d'halogénure de métal.

FIG. 1

FIG. 2

FIG. 3

FIG. 4

FIG. 5

FIG. 6

Action spectrum for the inactivation of IPNV with UV light

| 220 | 17.04808 |
|-----|----------|
| 239 | 2.423077 |
| 254 | 1 |
| 260 | 1.721154 |
| 280 | 3.134615 |

FIG. 7

FIG. 8

## Gesamtstrahlungsfluss (Total radiant power)

| | | |
|---|---|---|
| UVges (200-400) | 1100 W | 1072 W |
| UVA (315-400) | 277 W | 277 W |
| UVB (280-315) | 246 W | 243 W |
| UVC (200-280) | 592 W | 566 W |
| UVV (400-440) | 359 W | 361 W |
| TOC (200-230) | 190 W | 172 W |
| Germ 200-300 (200-300) | 675 W | 648 W |
| Germ 240-300 (240-300) | 419 W | 413 W |
| Germweighted 200-300 (200-300) | 617 W | 592 W |
| Germweighted 240-300 (240-300) | 385 W | 380 W |

## Normierter Strahlungsfluss (norm. Radiant power)

| | | |
|---|---|---|
| UVges (200-400) | 100% | **97%** |
| UVA (315-400) | 100% | 100% |
| UVB (280-315) | 100% | 99% |
| UVC (200-280) | 100% | 96% |
| UVV (400-440) | 100% | 101% |
| TOC (200-230) | 100% | 91% |
| Germ 200-300 (200-300) | 100% | 96% |
| Germ 240-300 (240-300) | 100% | 99% |
| Germweighted 200-300 (200-300) | 100% | 96% |
| Germweighted 240-300 (240-300) | 100% | 99% |

FIG. 9

FIG.10

**EP 2 969 966 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ANAT LAKRETZ ; ELIORA Z. RON ; HADAS MA-MANE.** Biofouling control in water by various UVC wavelengths and doses. *Biofouling,* April 2010, vol. 26 (3), 57-267
- **BOLTON JR ; LINDEN KG.** Standardization of methods for fluence (UV dose) determination in bench-scale UV experiments. *J Environ Eng ASCE,* 2003, vol. 129, 209-215
- **L. J. REED ; H. MUENCH.** A Simple Method of Estimating Fifty Per Cent Endpoints. *The American journal of Hygiene,* May 1938, vol. 27 (3), 493-497
- Ultraviolet Disinfection Guidance Manual 2006. ULTRAVIOLET DISINFECTION GUIDANCE MANUAL. 07 April 2006
- **H. LILTVED et al.** High resistance of fish pathogenic viruses to UV irradiation and ozonated seawater. *Aquacultural Engineering,* 2006, vol. 34, 72-82